# EUROPEAN PATENT APPLICATION

(11) **EP 2 543 386 A1**
(43) Date of publication of application: **09.01.2013**
(21) Application number: 11172622.0
(22) Date of filing: 05.07.2011
(51) Int. Cl.: A61K 39/00

(54) **Means and methods for active cellular immunotherapy of cancer by using tumor cells killed by high hydrostatic pressure**

(71) Applicant: Sotio a.s., Jinocany 252 25 Prague West (CZ)
(72) Inventor: Bartunková, Jirina, 110 00 Praha 1 (CZ); Spísek, Radek, 148 00 Praha 4 (CZ)
(74) Representative: Keller, Günter

(57) **Abstract**

Disclosed are pharmaceutical compositions for inducing an immune response against tumor cells comprising tumor cells which are made apoptotic by treatment with high hydrostatic pressure and dendritic cells, and methods for producing such compositions.

## Description

### Background of the present invention

Diseases caused by different tumors are still major problems in medicine and human health. The combination of surgery, chemotherapy and radiotherapy greatly improved the prognosis of cancer patients. Despite that this approach results frequently in a significant reduction of tumor mass, a small population of precursor tumor cells or cancer stem cells often survives and subsequently gives rise to a new population of tumor cells that leads to a relapse. Even if the main tumor is removed by surgical and/or other treatments minor amounts of circulating tumor cells may cause metastatic tumors in different areas of the body. Therefore, there exists a permanent need for alternative medicaments and methods of treatment which may be used alone or preferably be combined with other methods of tumor treatment.

The present invention relates to pharmaceutical compositions which can be used in tumor vaccination causing the body to produce an immunogenic reaction against tumor cells.

Tumor cells killed by standard modalities such as irradiation are normally non-immunogenic. If used for the generation of cancer immunotherapy products, irradiated tumor cells need to be administered in combination with a potent adjuvant. When used for pulsing of antigen presenting cells, such as dendritic cells, irradiated killed tumor cells do not provide an activating signal. Dendritic cells thus need to be activated by another substance, such as pathogen derived molecules.

A novel process is disclosed that induces an immunogenic death of human tumor cells, in particular ovarian and prostate cancer cells and acute lymphoblastic leukemia cells. Tumor cells killed by high hydrostatic pressure provide a potent activation stimulus to dendritic cells even in the absence of additional stimuli. Tumor cells killed by this method express high levels of immunogenic cell death markers and dendritic cells loaded with those immunogenic tumor cells induce high numbers of tumor specific T lymphocytes without expanding undesirable regulatory T lymphocytes.

Tumor cells are not or only weakly immunogenic and they usually do not have the capacity to induce a tumor specific immune response if used in the absence of a powerful adjuvant. Recent studies have shown that tumor cells killed by some chemotherapeutics, such as bortezomib, oxaliplatin and anthracyclines, can induce a tumor-specific immune response. This immunogenic cell death is characterized by molecular events shared for all described chemotherapeutics. Within hours after the initiation of immunogenic cell death, preapoptotic tumor cells translocate calreticulin and heat shock proteins from the endoplasmic reticulum to the cell surface together with other molecules that serve as 'eat me' signals (phosphatidylserine).

At the same time, tumor cells undergoing immunogenic tumor cell death downregulate the expression of 'don't eat me' signals (such as surface CD47) to facilitate tumor-cell recognition and engulfment by dendritic cells. Additionally, following permeabilization of the plasma membrane, cells release the late apoptosis marker high mobility group box 1 (HMGB1) into the extracellular milieu. HMGB1 can bind several pattern recognition receptors (PRRs), such as Toll-like receptor 2 (TLR2), TLR4 and receptor for advanced glycosylation end products (RAGE). The release of this protein seems to be required for optimal presentation of antigens from dying tumor cells, T-cell priming by dendritic cells and subsequent T-cell-mediated elimination of the tumor.

Use of tumor cells killed in such a way that they become immunogenic is extremely important for the design of cancer immunotherapeutic strategies. Administration of immunogenic tumor cells can induce a tumor specific immune response that will then control the growth of tumor cells. This will slow down or even stabilize the progression of the disease and improve the prognosis of cancer patients. It is also assumed that the distribution of tumor cells circulating in the body and the formation of metastases can be at least substantially reduced.

### Preferred embodiments of the present invention

A novel method and pharmaceutical compositions are disclosed that induce an immunogenic cell death of human tumors, in particular ovarian and prostate cancer cells and acute lymphoblastic leukemia cells to a much higher extent than recently described chemotherapeutics. Tumor cells killed by this method express high levels of immunogenic cell death markers and induce high numbers of tumor specific T lymphocytes without inducing regulatory T cells that could inhibit anti-tumor immune response.

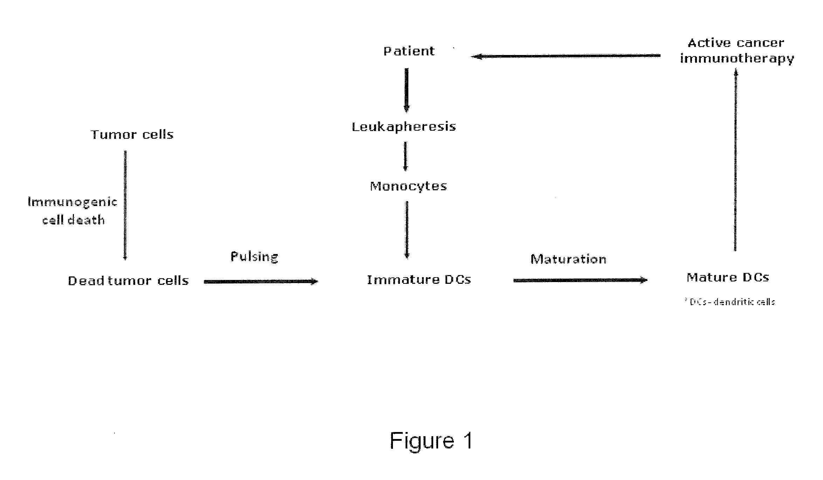

The general principle of a preferred cancer immunotherapy protocol based on the administration of mature dendritic cells (DCs) loaded with killed tumor cells is shown in Figure 1. All steps of the generation of the final pharmaceutical composition are performed under Good Manufacturing Practice conditions in GMP facility.

The first step in the process of generation of the pharmaceutical composition for each patient is a leukapheresis performed for the purpose of collecting large numbers of monocytes from the peripheral blood. In a preferred embodiment the leukapheretic product is then diluted in a suitable buffer, such as PBS+1mM EDTA (Lonza, Vierviers, Belgium) and mononuclear cells are separated by Premium Ficoll Paque (GE Healthcare, Little Chalfont, UK) gradient centrifugation. Collected mononuclear cells (PBMC) are then washed [e.g. in PBS+1mM EDTA (Lonza)], resuspended in Cell Gro medium and plated in triple flasks (e.g. NUNC, Roskilde, Denmark) at 1x10⁶ cells per cm² of surface area. After two hours non-adherent cells are washed with PBS (Lonza). Adherent monocytes are cultured for 6 days in Cell Gro medium with 20 ng/ml of IL-4 (Gentaur) and 500 U/ml of GM-CSF (Gentaur), fresh cytokines are added on day 3.

Immature DCs are harvested on day 6 and loaded with killed tumor cells (e.g. prostate cancer cell line, ovarian cancer cell line, acute lymphoblastic leukemia cell line). Freshly thawed, immature DCs (day 6) are fed tumor cells at a fixed DC: tumor cell ratio of 5:1 for 4h. Tumor cell-pulsed DCs are then matured by 25 µg/ml of Poly I:C during overnight incubation and cryopreserved and stored in liquid nitrogen. Before administration, 1x10⁷ mature DCs pulsed with tumor cells are resuspended in 0,9% NaCl (Baxter) and injected subcutaneously in the inguinal and brachial area within 12 hours preferably from 30 minutes up to 12 hours. Administration of this form of cancer immunotherapy is preferably repeated in regular intervals of 2-6 weeks in order to continuously boost the immune response.

The above-described preferred embodiment is, however, in no way limiting. In the broadest scope the invention can be performed in alternative ways depending on the specific needs of tumor treatment. The above-identified preferred embodiment describes the invention by using autologous cells whereby the tumor cells are obtained from the patient to be treated and the dendritic cells are also obtained from the patient to be treated.

In a more general way, however, the present invention relates to a pharmaceutical composition for inducing an immune response against tumor cells comprising
a) tumor cells which are apoptotic, whereby apoptosis is caused by treatment with hydrostatic pressure and
b) dendritic cells.

It is an important aspect of the present invention that the tumor cells which are used in the pharmaceutical composition are apoptotic cells and not necrotic cells. The person skilled in the art is aware of the differences between apoptosis versus necrosis. Cell death and subsequent post-mortem changes, called necrosis, are integral parts of normal development and maturation cycle. Despite the importance of this process the mechanism underlying cell death are still poorly understood although there are several publications relating to the mechanisms which occur when a cell is dying. Apoptosis in the sense of the present invention is understood as a programmed, managed form of cell death whereby necrosis is an unordered and accidental form of cellular dying.

In the present invention apoptosis is understood as a mode of cell death that occurs under normal physiological conditions and the cell is an active participant of its own demise. Cells undergoing apoptosis show characteristic morphological and biochemical features. These features include chromatine aggregation, nuclear and cytoplasmatic condensation, partition of cytoplasm and nucleus into membrane-bound vesicles (apoptotic bodies) which contain ribosomes, morphologically intact mitochondria and nuclear material. Since these apoptotic bodies are in vivo normally recognized and phagocytized by either macrophages or adjacent epithelial cells it is important that the tumor cells used in the present method resemble as close as possible apoptotic tumor cells. Apoptosis is usually limited to individual cells and does not cause inflammatory responses.

Necrosis on the other hand occurs when cells are exposed to extreme physiological conditions which may result in damage to the plasma membrane. Necrosis begins with an impairment of the cells' ability to maintain hemostasis, leading to an influx of water and extracellular ions. Intracellular organelles, most notably the mitochondria and the entire cell swells and ruptures. Due to the ultimate breakdown of the plasma membrane the cytoplasmic contents, including lysosomal enzymes, are released into the extracellular fluid. Therefore, in vivo necrotic cell death is often associated with extensive tissue damages resulting in an intense inflammatory response. It is important that the tumor cells used in the pharmaceutical compositions are apoptotic and not necrotic.

According to the present invention the apoptotic cells are produced by a treatment with high hydrostatic pressure. A high hydrostatic pressure as understood in the present invention is defined as a pressure head equal to or greater than 100 MPa [1 MPa = 10 bar = 9.86923 atm = 145.0377 psi]. High hydrostatic pressure can be produced by an equipment which is for example described in Weiss et al., Journal of Immunotoxicology, 2010, pp 194-209, in particular in Figure 1.

The high hydrostatic pressure treatment of the tumor cells is preferably performed in a pressure autoclave. The tumor cells are placed in suitable cryogenic vials which are filled completely with cell suspension and closed tightly whereby the appearance of air bubbles has to be avoided. Afterwards the vials are sealed with a flexible film (e.g. parafilm^{®}) and the prepared vials are placed in the pressure chamber which is filled with a pressure transmitting medium. Afterwards the high pressure is produced by a suitable device and the cells are maintained for a sufficient time under such high pressure.

In a preferred embodiment the hydrostatic high pressure is maintained for at least 10 minutes at a pressure of at least 200 MPa. In a more preferred embodiment the tumor cells are maintained for a time range of 10 minutes to 12 hours, preferably 10 minutes to 1 hour and especially preferred 10 to 30 minutes at a pressure in the range of 200-300 MPa, preferably 200-250 MPa.

The tumor cells to be used in the pharmaceutical composition can be derived from different sources. In one particular embodiment the tumor cells are derived from a primary tumor or from a metastatic tumor of the patient to be treated. The tumor cells can be obtained by biopsy or surgery. The tissue is disintegrated and the separated and purified tumor cells can be used immediately. It is also preferred to establish a cell line of the primary tumor and to use the so obtained cells for tumor vaccination. Alternatively the tumor cells may be obtained from suitable tumor cell lines. Such tumor cell lines may be prepared from the autologous tumor. Alternatively, tumor cells may be used which are commercially available from depository institutions like for example ATCC.

The other components of the pharmaceutical composition are dendritic cells, preferably immature dendritic cells. It is known that there are three main types of antigen-presenting cells in the peripheral lympoid organs that can activate T cells, namely dendritic cells, macrophages and B cells. The most potent of these are dendritic cells whose known function is to present foreign antigens to T cells. Immature dendritic cells are located in tissues throughout the body, including the skin, gut and respiratory tract. Dendritic cells exist in two functionally and phenotypically distinct stages, immature and mature dendritic cells. Immature dendritic cells have high endocytic activity, are specialized in antigen capture and processing and reside in peripheral tissues in vivo. Immature dendritic cells play a crucial role in the induction and maintenance of peripheral tolerance. Upon exposure to pathogen-derived products or innate pro-inflammatory signals, dendritic cells lose their phagocytic activity and migrate to draining lymph nodes while becoming mature dendritic cells. Mature dendritic cells have a high antigen-presenting capability and T-cell stimulatory capacity due to the expression of high levels of antigen-presenting, adhesion and co-stimulatory molecules as well as other dendritic cell-specific markers such as CD83 and DC-LAMP. DC-LAMP (DC-lysosome-associated membrane glycoprotein), also known as LAMP-3 (lysosomal-associated membrane protein 3), TSC-403 or CD208, is a 416 amino acid lysosome membrane protein that belongs to the LAMP family. DC-LAMP is expressed in lung, lymphoid organs and dendritic cells, and is upregulated in carcinomas of the esophagus, colon, rectum, ureter, stomach, breast, fallopian tube, thyroid and parotid tissues.

The immature dendritic cells to be used in the pharmaceutical composition may be obtained from different sources. In a preferred embodiment the immature dendritic cells are obtained from the patient to be treated. Alternatively, however, the immature dendritic cells may be obtained from other sources such as commercially available blood products like plasma obtainable from blood collecting agencies.

For the preparation of a pharmaceutical composition according to the present invention suitable immature dendritic cells are prepared. Dendritic cells (DC) can be prepared by different methods and may exhibit different properties. In a preferred embodiment of the present invention dendritic cells are obtained from monocytes isolated by leukapheresis. DC comprise less than 1% of mononuclear cells in the peripheral blood. Leukapheresis can be used to isolate approximately 10⁶ to 10⁷ dendritic cells and may be combined with positive or negative selection techniques. While the direct isolation of dendritic cells from peripheral blood allows rapid preparation of dendritic cells it may require repeated leukapheresis if multiple immunizations are required in a protocol.

An alternative and particularly preferred method of preparing dendritic cells is to generate them ex vivo. Monocytes are dendritic cell precursors which may be enriched from peripheral blood mononuclear cells by techniques such as leukapheresis, plastic adherence, density gradient centrifugation, positive selection of CD14+ cells, negative selection of B- and T-cells and combinations thereof. DC may be cultivated and differentiated by treating an enriched precursor cell population for approximately 5-7, preferably 7 days with cytokines, in particular with granulocyte macrophage-colony stimulating factor (GM-CSF) + interleukin 4 for 5 days or with interleukin 13. An advantage of this embodiment is that more than 10⁹ DC may be prepared from a single leukapheresis product and such a preparation may be used for multiple further vaccinations by deep freezing the DC preparation preferably in liquid nitrogen. While DC may be cultured in a variety of media it is preferred to use either serum-free media or media containing autologous serum. For the industrial preparation of the pharmaceutical composition it is particularly preferred to prepare the immature dendritic cells in a large scale in such a manner that the occurrence of anaphylactic reactions (e.g. due to fetal calf serum) or the contamination of viruses is avoided.

In the next step of the preparation of the pharmaceutical composition the immature dendritic cells are loaded with the apoptotic tumor cells which are obtained by treatment with high hydrostatic pressure. In a preferred embodiment the immature dendritic cells which were brought into contact with the apoptotic tumor cells are matured by using a variety of stimuli such as the addition of Tumor Necrosis Factor α (TNF-α) or lipopolysaccharide or poly I:C.

The obtained pharmaceutical composition can be preserved for the administration, preferably by cryopreservation. The cryopreservation of biospecimen is widely practiced in clinical medicine and biomedical research. However, the impact of this process on cell viability and particularly function sometimes may be underestimated. Therefore, the used method of freezing of the cell preparation prior to use in cancer vaccine should be viewed with caution. The effect of cryopreservation certainly depends on the specific cells used and it has to be examined whether the biological activity of the pharmaceutical composition is altered by the cryopreservation. It may be required to add protective components like non-immunogenic polysaccharides.

The pharmaceutical composition of the present invention can be administered intravenously (IV), intradermally, subcutaneously or intralymphatically whereby subcutaneously is particularly preferred.

The optimal dose and frequency of immunization of the pharmaceutical composition depends on the type of tumor, the age and condition of the patient and the stage of progression of the tumor disease. In a preferred embodiment there is first applied an immunizing dose of the pharmaceutical composition which can be followed by long-term administration of 2 to 4 booster injections applied in intervals ranging from 2 to 6 weeks.

Although it is known in the art to use adjuvant agents in tumor therapy vaccination it is preferred in the course of the present invention not to use any further adjuvant such as lipopolysaccharide, incomplete Freund's adjuvant or heat shock proteins.

It is an important aspect of the present invention that the tumor cells treated with high hydrostatic pressure are in such a stage that they cannot grow and form a metastatic tumor after application to the patient. Therefore, the tumor cells treated with high hydrostatic pressure may for safety reasons additionally be treated by high energy radiation which is also used for the treatment of tumor patients. The radiation therapy uses high energy radiation which kills the tumor cells by damaging their DNA. It is, however, important not to change the surface structure or the form of the apoptotic tumor cells by the radiation treatment. The dose of radiation must be carefully determined by finding out the narrow range wherein the immunological properties are maintained, but the formation of new metastases is avoided.

The pharmaceutical composition as described herein before can be used for the treatment of a human by cancer immunotherapy (vaccination). The tumor cells which can be derived from a patient to be treated are brought to an apoptotic stage with the high hydrostatic pressure treatment described above. Immature dendritic cells are obtained preferably by leukapheresis from the same patient and the cells are amplified ex vivo by treatment with cytokines. A suitable amount of such immature dendritic cells (e.g. 10⁷-10⁸ cells) is loaded with the apoptotic tumor cells whereby the optimal range of immature dendritic cells : apoptotic tumor cells is 10:1 1 to 1:1, preferably 5:1 to 3:1. After maturation of the dendritic cells the pharmaceutical composition can be applied to the patient.

According to the present invention the following materials and methods are preferably used:

It has been shown that a treatment of ovarian and prostate cancer cells and acute lymphoblastic leukemia cells by 10 min with high hydrostatic pressure (200MPa) at about 21°C leads to the induction of an immunogenic cell death of tumor cells. Tumor cells killed by HHP (high hydrostatic pressure) are immunogenic to much higher extent than tumor cells killed by anthracyclines, the only cytostatics known to induce immunogenic cell death. HHP-killed immunogenic tumor cells are avidly phagocytosed by antigen presenting cells and induce their maturation event in the absence of additional pathogen-derived stimuli, such as LPS. Antigen presenting cells loaded with HHP killed tumor cells induce a robust CD4 and CD8 mediated tumor specific T cell responses and do not induce potentially harmful regulatory T cells. HHP killed tumor cells thus represent a powerful tool for clinical cancer immunotherapy approaches.

Despite the continuous introduction of new drugs and further improvements of chemotherapy protocols, it is likely that, at some point, chemotherapy will reach its limits, and clinical efficacy will plateau. Moreover, despite the undeniable success in the treatment of some malignancies, in some tumors, particularly in solid tumors, chemotherapy is rarely curative. A combination of treatment modalities has been a standard strategy for cancer treatment, the combination of surgery with chemo- or radiotherapy being a classical example. Effort should be made not only to design modern immunotherapeutic strategies but also to incorporate immunotherapy approaches into current chemotherapy protocols. Chemotherapy and immunotherapy should not be henceforth considered antagonist forms of therapy, and it is conceivable that their rational combination will substantially improve the prognosis of cancer patients.

The following materials and methods were preferably used:

Preferred cell lines: Acute lymphoblastic leukemia cell lines,(REH, DSMZ, Braunschweig, Germany), ovarian cancer cells (OV90, ATCC, Teddington, UK), prostate cancer cells (LNCap, ATCC, Teddington, UK) were used. All cell lines were cultured in RPMI 1640 medium (Gibco). All media were supplemented with 10% heat-inactivated fetal bovine serum (Lonza), 100 U/ml penicillin and 2 mmol/L L-glutamine.

Isolation of primary tumor cells: Primary ovarian and prostate cancer cells were obtained from patients undergoing surgery. Leukemic blasts from patients with acute lymphoblastic leukemia were obtained from the bone marrow of (ALL) patients by gradient centrifugation on Ficoll gradient

Apoptosis induction and detection: Tumor cell death was induced by 10min treatment with high hydrostatic pressure. Cell death was assessed by annexin V fluorescein isothiocyanate staining. Briefly, 2x10⁵ cells per sample were collected, washed in PBS, pelleted, and resuspended in an incubation buffer containing annexin V fluorescein isothiocyanate antibody. The samples were kept in the dark and incubated for 15 min before the addition of another 400 µl of 0,1% propidium iodide incubation buffer and subsequent analysis on an Aria fluorescence-activated cell sorter (BD Bioscience) using FlowJo software.

Flow cytometric analysis of hsp70, hsp90 and CRT (calreticulin) on the cell surface: A total of 10⁵ cells were plated in 12-well plates and treated the following day with the indicated agents or were - as a control - UV-irradiated (7,6 J/cm²) for 6, 12 or 24 h or were treated for 10min with high hydrostatic pressure at 21 degrees centigrade's. The cells were collected and washed twice with PBS. The cells were incubated for 30 min with primary antibody diluted in cold blocking buffer (2% fetal bovine serum in PBS), followed by washing and incubation with the Alexa 648-conjugated monoclonal secondary antibody in a blocking solution. Each sample was then analyzed by FACScan (BD Bioscience) to identify cell surface hsp70, hsp90 and CRT.

Detection of HMGB1 release: HMGB1 enzyme-linked immunosorbent assay II kits were obtained from SHINO-TEST CORPORATION (Tokyo, Japan). REH cells, OV90 cells, LNCap cells, primary ovarian cells and leukemic blasts (10⁶) were plated in 1 ml full medium appropriate for the cell type. Supernatants were collected at different time points, dying tumor cells were removed by centrifugation, and the supernatants were isolated and frozen immediately. Quantification of HMGB1 in the supernatants was assessed by enzyme-linked immunosorbent assay according to the manufacturer's instructions.

Fluorescent microscopy: Immunofluorescence: For surface detection of CRT, the cells were placed on ice, washed twice with PBS and fixed in 0,25% paraformaldehyde in PBS for 5 min. The cells were then washed twice in PBS, and a primary antibody diluted in cold blocking buffer was added for 30 min. After two washes in cold PBS, the cells were incubated for 30 min with the appropriate Alexa 648-conjugated secondary antibody. The cells were fixed with 4% paraformaldehyde for 20 min, washed in PBS for 20 min and mounted on slides.

For phagocytosis, the DCs were stained with Vybrant® DiO cell labeling solution (Invitrogen). The tumor cells were stained with Vybrant® Dil cell labeling solution (Invitrogen) and cultured in the presence of anthracyclins, UV light irradiation or 10 min treatment with high hydrostatic pressure at 21 degrees centigrade's . Immature DCs (day 5) were fed tumor cells at a DC/tumor cell ratio of 1:5. The cells were fixed with 4% paraformaldehyde for 20 min, washed in PBS for 20 min and mounted on slides with ProLong Gold antifade reagent (Invitrogen).

Generation of tumor-loaded DCs and induction of tumor cell death: DCs were generated by culture of purified CD14⁺ cells isolated from buffy coats in the presence of granulocyte-macrophage colony-stimulating factor (GM-CSF) (Gentaur, Brussels, Belgium) and interleukin-4 (IL-4) (Gentaur, Brussels, Belgium). Tumor cells were killed by 10 min. treatment with high hydrostatic pressure at 21 degrees centigrade's, or - as controls - by UV irradiation or by anthracyclines. The extent of apoptosis was monitored by annexin V/Pl staining. The cells were extensively washed prior to feeding to DCs. Immature DCs (day 5) were fed tumor cells at a DC/tumor cell ratio of 1:5. In some experiments, pulsed DCs were stimulated with 100 ng/ml of lipopolysaccharide (LPS) (Sigma) for 12 h.

FACS analysis of DC phenotype after interaction with killed tumor cells: The phenotype of DCs cultured with tumor cells was monitored by flow cytometry. Tumor cells were killed by a selected cytostatic agent or UV irradiation (comparative examples) or 10 min treatment with high hydrostatic pressure at 21 degrees centigrades (according to the present invention) and were cocultured for 24 h with immature DCs. For some experiments, the DCs and tumor cells were dye-labeled before coculture to monitor phagocytosis. Monoclonal antibodies (mAbs) against the following molecules were used: CD80-FITC, CD83-FITC, CD86-PE, CD14-PE (Immunotech, Marseille, France), CD11c-PE, HLA-DR (BD Biosciences, San Jose, CA).

The DCs were stained for 30 minutes at 4°C, washed twice in phosphate-buffered saline (PBS) and analyzed using FACS Aria (BD Biosciences) using FlowJo software. The DCs were gated according to the FSC and SSC properties. The appropriate isotype controls were included, and 50000 viable DCs were acquired for each experiment.

Evaluation of IFN-γ producing tumor-specific T cells: Unpulsed or tumor cells-loaded DCs were added to autologous T cells at a ratio of 1:10 on days 0 and 7 of culture. IL-2 (25-50 international units/mL; PeproTech) was added on days 2 and 7 of culture. The cultures were tested for the presence of tumor-specific T cells 7 to 9 days after the last stimulation with DCs. The induction of tumor-reactive, interferon (IFN)-γ-producing T cells by tumor-loaded DCs was determined by flow cytometry. The T cells were stained with anti-human CD8/IFN-γ.(1) Frequency of regulatory T lymphocytes in the culture was analyzed by staining with CD4/CD25 and FoxP3. Regulatory T cells were identified by flow cytometry as CD4 positive, CD25 positive and FoxP3 positive.

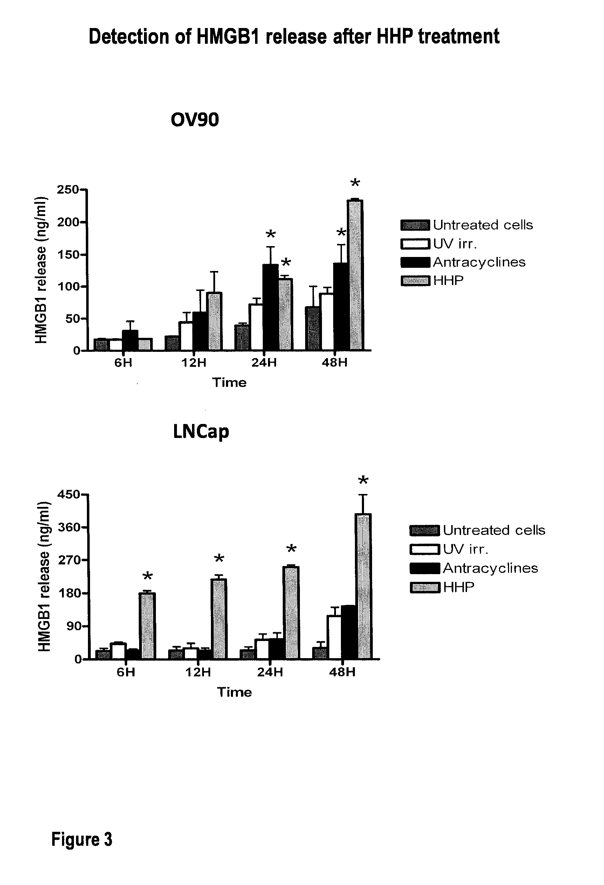

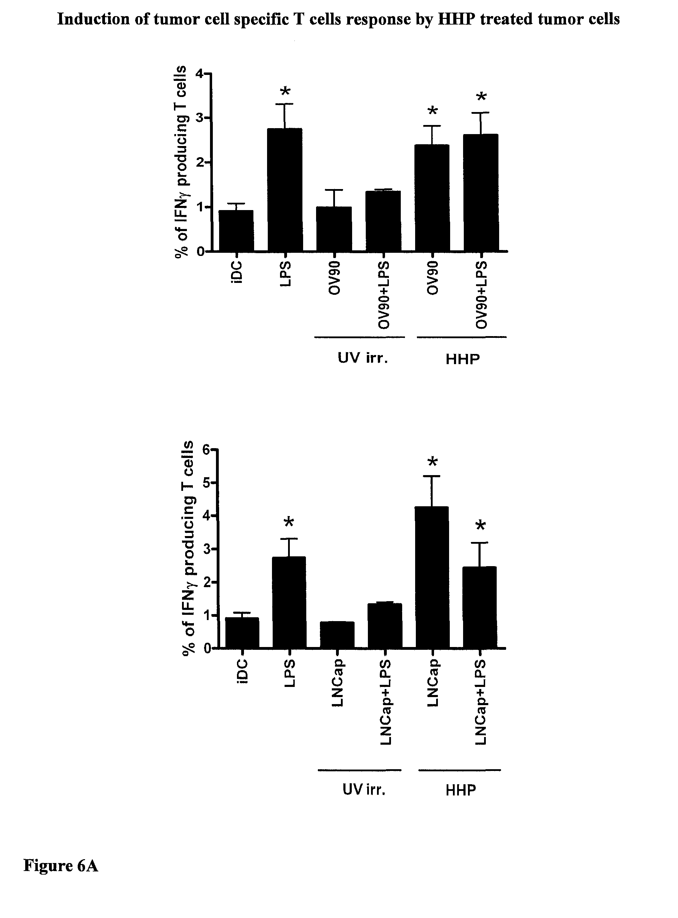

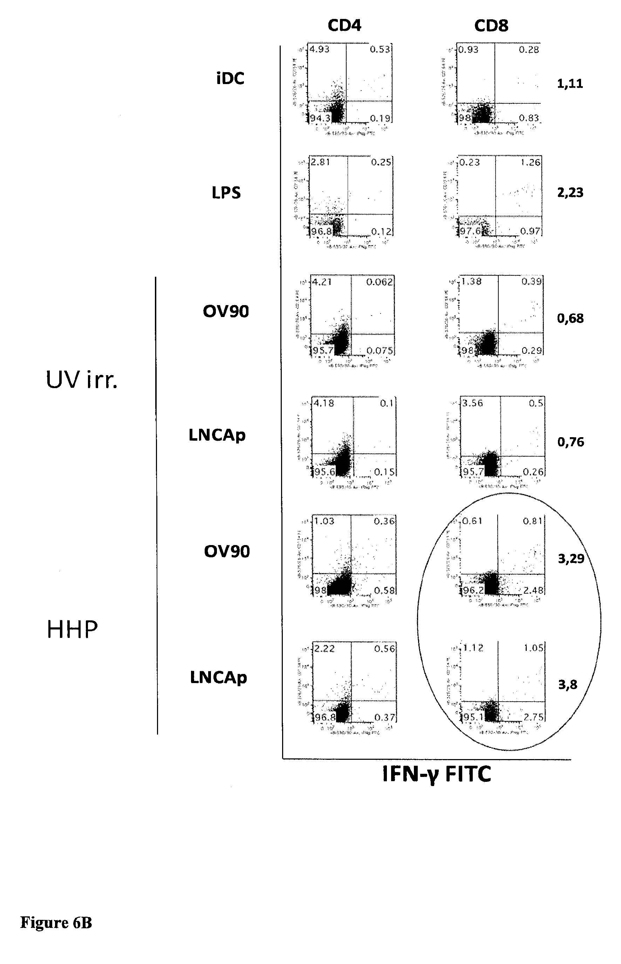

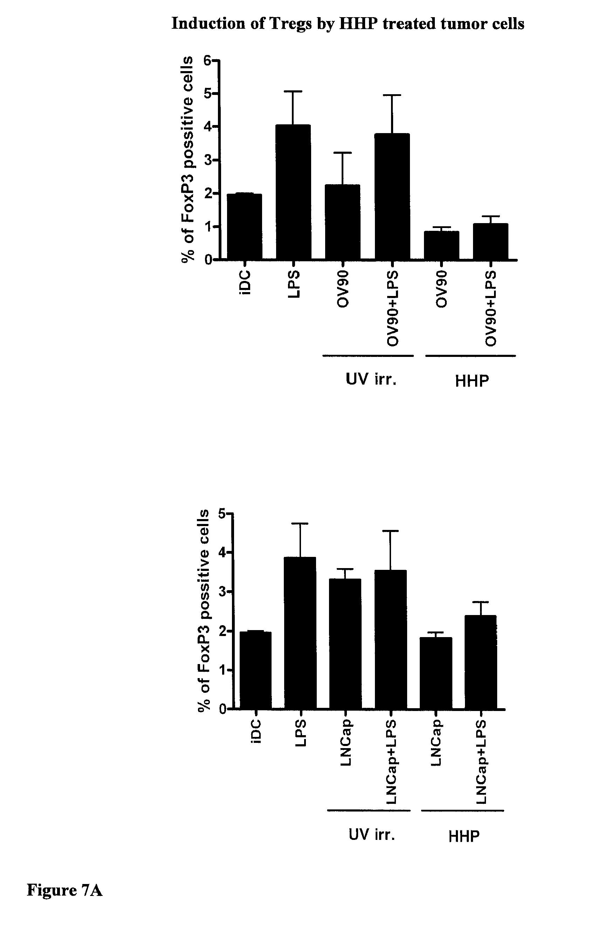

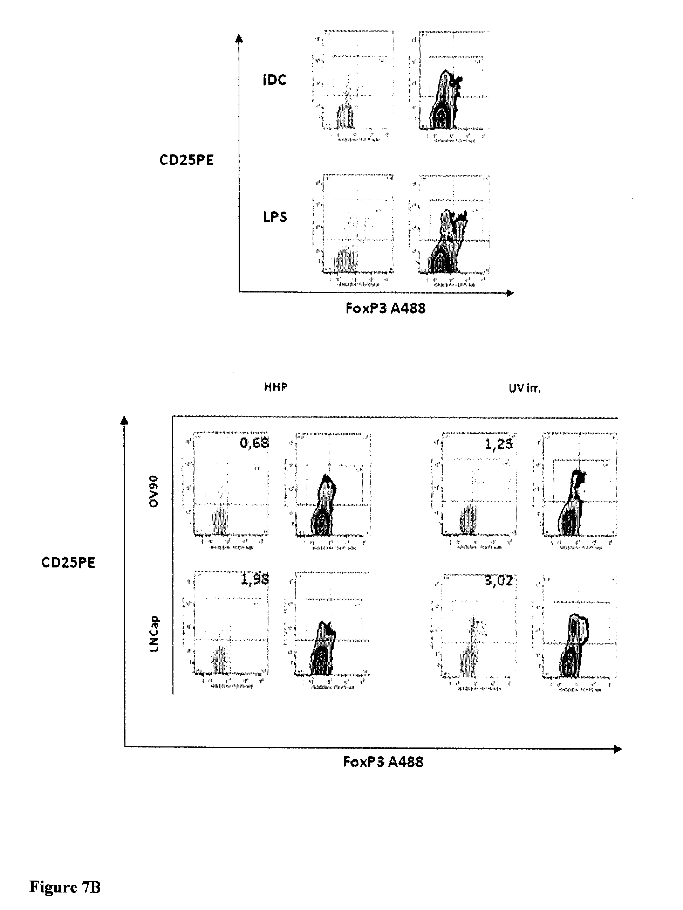

The invention and the results obtained by the experiments are illustrated by the Figures:
**Figure 1****:**
   The schematic drawing shows how a pharmaceutical composition of the present invention can be obtained. Tumor cells obtained either from the patient or from cell lines are treated with high pressure whereby the cells become apoptotic. Dendritic cells are isolated via leukapheresis. Immature dendritic cells and apoptotic tumor cells are combined whereby mature dendritic cells are produced which can be used as vaccine.
**Figure 2**
   High hydrostatic pressure induces the expression of heat shock proteins on human tumor cells. The summary of a total of 5 experiments is shown. * P value for comparison with irradiated tumor cells, P <0,05. The time dependent expression of the markers HSP70, HSP90 and calreticulin on two tumor cell lines (OV90 and LNCap) caused by different treatments is shown.
**Figure 3**
   High hydrostatic pressure induces the release of HMGB1 from treated tumor cells (OV90 and LNCap). The summary of a total of 5 experiments is shown. * P value for comparison with irradiated tumor cells, P <0,05. Figure 3 shows that concerning the time dependent release of HMBG1 the HHP treatment is much more effective than other conventional treatments.
**Figure 4**
   The kinetics of phagocytosis of high hydrostatic pressure treated tumor cells by immature DCs. Summary of 5 independent experiments (4a) and representative results (4b) are shown. HHP treatment is compared with UV treatment at 0°C and 37°C.
**Figure 5**
   The phenotype of dendritic cells based on the markers OD86 and HLA-DR after interaction with high hydrostatic pressure-killed tumor cells (OV90 and LNCap) is shown. Day 5 immature DCs were cultured for 24 h with tumor cells killed by HHP or irradiation. After 24 h, the expression of maturation associated molecules on DCs was analyzed by flow cytometry. LPS was used as control. The mean fluorescence intensity and representative histograms are shown. * P value for comparison with irradiated tumor cell-loaded DCs, P < 0.05.
**Figure 6**
   The induction of tumor-specific T cells by high hydrostatic pressure killed tumor cells (LNCap and OV90) is compared with UV irradiation. The data show a summary (Fig. 6A) and representative staining (Fig. 6B) of five independent experiments. * P value for comparison with irradiated tumor cells, P <0,05.
**Figure 7**
   The induction of regulatory T cells by high hydrostatic pressure killed tumor cells. The data show a summary (Fig. 7A) and representative staining (Fig. 7B) of five independent experiments. * P value for comparison with irradiated tumor cells, P <0,05.

The present invention is further illustrated by the following examples which are, however, not limiting:

### Example 1

### Expression of immunogenic cell death markers hsp70, hsp90 and calreticulin by human cancer cell lines and human primary tumor cells after the treatment with high hydrostatic pressure

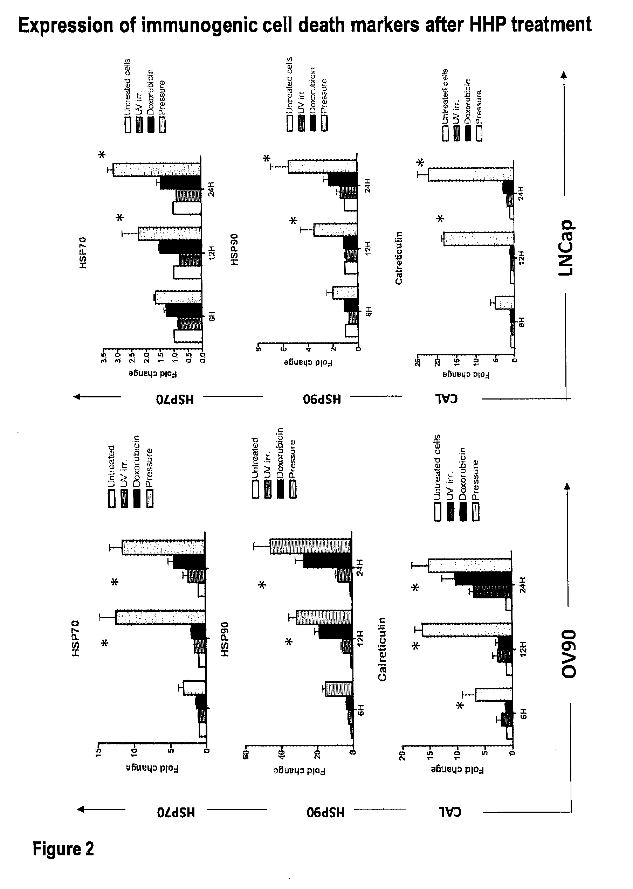

Leukemic, ovarian and prostate cancer cell lines and primary tumor cells were treated for 10min with high hydrostatic pressure (HHP, 200 MPa) at 21 degrees centigrade's and the expression of the known immunogenic cell death markers hsp70, hsp90 and calreticulin was monitored at 6, 12 and 24h. Significant expression of calreticulin, hsp70 and hsp90 was detected 6, 12 and 24h after HHP treatment for all tested tumor models. The expression of immunogenic molecules was significantly higher than the expression induced by anthracyclins, the only known inducers of immunogenic cell death (Figure 2). Increased expression of calreticulin and heat shock proteins after HHP treatment was accompanied by their translocation to the cell surface. HHP treatment also induced a rapid and substantial release of HMGB1, a soluble marker of immunogenic cell death. Release of HMGB1 was much higher than in the case of UV irradiation or anthracyclines. (Figure 3).

Maximal release of HMGB1 nuclear protein was detected 48h after the induction of tumor cell death.

### Example 2

### Treatment of tumor cells by high hydrostatic pressure increases their phagocytosis by antigen presenting cells

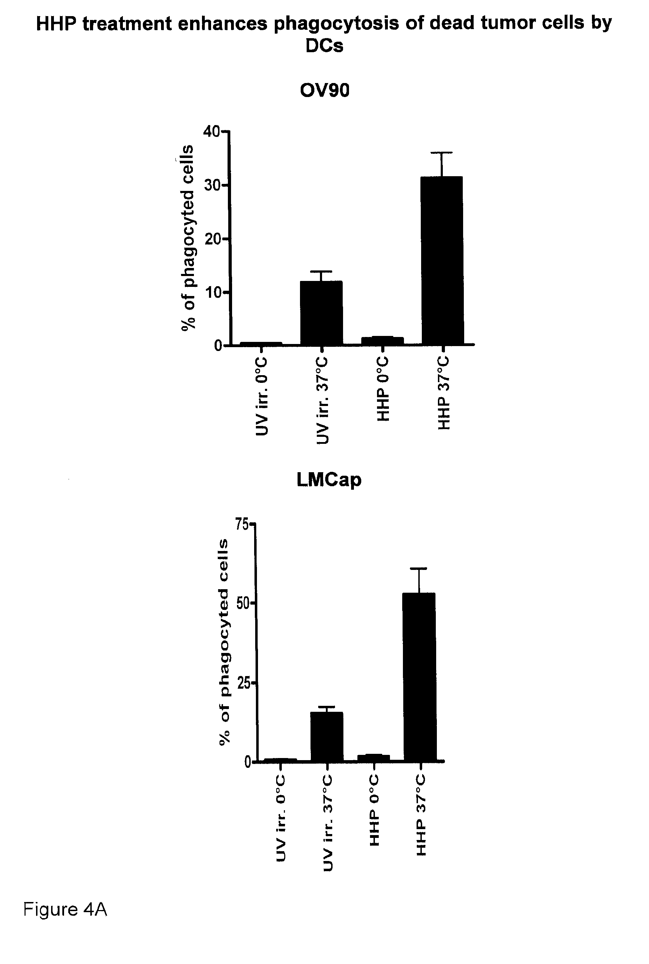

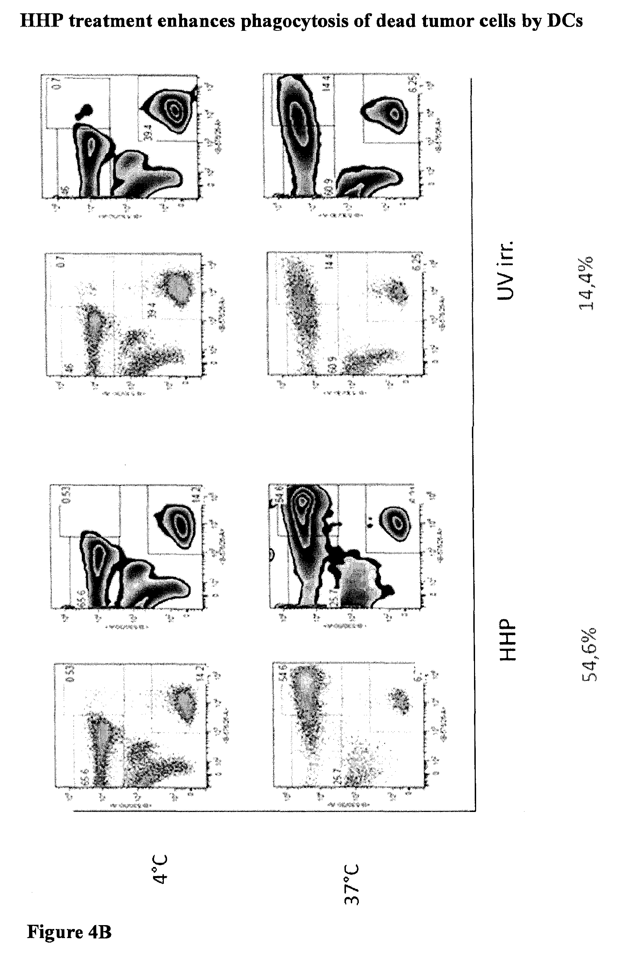

In view of the established role of calreticulin as an 'eat me' signal, we investigated the rate of phagocytosis of tumor cells killed by high hydrostatic pressure by dendritic cells (DCs), the most efficient antigen presenting cells that are crucial for the initiation of an immune response. High hydrostatic pressure treated tumor cells were phagocytosed at faster rate and to a higher extent than the tumor cells killed by other modalities, such as anthracyclines or UV irradiation. After 12 h, the extent of phagocytosis of leukemic cells treated with anthracyclines was 4-fold higher than of cells killed by UV irradiation or anthracyclines tested drugs. (Figures 4a and 4b).

### Example 3

### Phagocytosis of high hydrostatic pressure-treated tumor cells induces the maturation of DCs

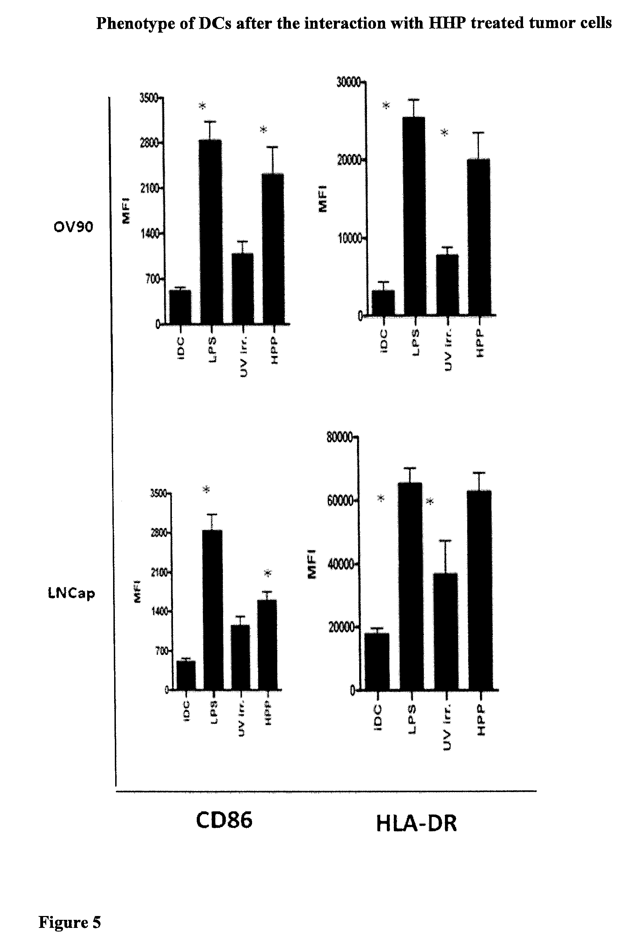

The ability of DCs to activate the immune response depends on their activation status and the expression of costimulatory molecules. In normal circumstances the most efficient maturation of DCs is induced by molecules derived from pathogens, such as lipopolysacharide (LPS) from Gram negative bacteria. Only activated (mature) DCs that express high levels of costimulatory molecules can initiate the immune response. We analyzed the phenotype of DCs that phagocytosed tumor cells killed by the HHP. The interaction of DCs with HHP-treated tumor cells induced the upregulation of costimulatory molecules (CD86, CD83) and maturation associated molecules (HLA-DR) to a similar extent as activation by LPS (Figure 5). Thus tumor cells killed by HHP can induce DCs maturation comparable to pathogen derived LPS.

### Example 4

### DCs presenting high hydrostatic pressure treated tumor cells induce tumor-specific T cells and induce low numbers of inhibitory regulatory T cells

To investigate whether tumor cells treated with HHP and expressing immunogenic cell death markers induce anti-tumor immunity, we evaluated the ability of tumor cell-loaded DCs to activate tumor cell-specific T cell responses. Tumor cells killed by HHP were cocultured with immature DCs with or without subsequent maturation with LPS. These DCs were then used as stimulators of autologous T cells, and the frequency of IFN-γ-producing T cells was analyzed one week later after restimulation with tumor cell-loaded DCs. DCs pulsed with HHP killed tumor cells induced a greater number of tumor-specific IFN-γ-producing T cells in comparison with DCs pulsed with irradiated cells, even in the absence of additional maturation stimulus (LPS) (Figures 6a and 6b).

Additionally, we also tested the frequency of regulatory T cells (Tregs) induced in DC and T cell cocultures. Induction of Tregs is undesirable in the case of tumor immunotherapy as Tregs inhibit the immune response directed against the tumor. DCs pulsed with tumor cells killed by HHP had a lower capacity to expand regulatory T cells when compared with both immature DCs and LPS-activated DCs (Figures 7a and 7b).

### Example 5

Active cellular immunotherapy can be administered as a single treatment modality in the case of minimal residual disease after primary treatment of the tumor by surgery or radiotherapy. In prostate cancer it may concern patients with signs of biochemical relapse (increasing levels of prostate-specific-antigen PSA in the peripheral blood measured by ultrasensitive method).

The best results of the present invention can be obtained when the primary tumor is removed from the patient by surgery. The pharmaceutical composition as described in the present application can be produced from the tumor cells which have been isolated from the tumor tissue or from tumor cell lines.

A patient (68 years old) suffering from prostate cancer was diagnosed at an early stage of the tumor development. Tumor was removed but few months after the surgery rising levels of PSA were detected. Patients thus underwent leukapheresis and immature dendritic cells were differentiated from isolated monocytes. Tumor cells from the prostate cancer cell line were rendered apoptotic treatment with high hydrostatic pressure as described herein and the apoptotic tumor cells were brought into contact with the immature dendritic cells in order to prepare the vaccine composition.

The pharmaceutical composition was divided into aliquots that were frozen in the liquid nitrogen until use. The first application of the tumor vaccination occurred 4 weeks after surgery had taken place. Booster applications followed at 6, 8 and 10 weeks after surgery.

By the vaccination an immune response against the small number of surviving tumor cells has lead to a substantial slowing down of regrowth of tumor cells and resulted in the prolongation of the survival of the patient.

### Example 6

In advanced cancer patients, active cellular immunotherapy should be combined with chemotherapy (i.e. docetaxel in prostate cancer) according to the concept of chemo-immunotherapy.

A patient (76 years old) suffering from advanced prostate cancer was treated according to the present invention. The usual chemotherapy was combined with the active cellular immunotherapy as disclosed herein. The patient has been treated at the age of 65 years with prostate tumor. After removal of the tumor by surgery and hormone treatment the level of PSA (prostate specific antigen) was kept at a low level showing that the prostate cancer cells did not grow. After 12 months of hormone therapy metastatic prostate cancer developed at several positions in the body (in particular in the bones) and the tumor became hormone refractory. The patient was approved for the treatment of hormone refractory prostate cancer with docetaxel in combination with active cellular immunotherapy based on dendritic cells.

Before the chemotherapy started, immature dendritic cells were generated from monocytes obtained during leukapheresis. Tumor cells from prostate cancer cell lines were treated with hydrostatic pressure for 30 minutes at a pressure of 210 MPa at 21°C. 10⁹ tumor cells treated according to the present invention were used to pulse 10⁹ immature dendritic cells and aliquots of the mature dendritic cells which have been pulsed before with those tumor cells were deep-frozen in liquid nitrogen and used for later applications.

Phases of remission of the patient after treatment with cytostatic agents were used for applying the anti-tumor vaccine of the present invention. The vaccination and booster applications were used three times and the growth of metastatic tumor cells could be slowed down substantially.

## Claims

1. Pharmaceutical composition for inducing an immune response against tumor cells comprising
a) tumor cells which are apoptotic caused by treatment with high hydrostatic pressure and
b) dendritic cells.

2. Pharmaceutical composition according to claim 1, **characterized in that** the tumor cells were treated with hydrostatic pressure for at least 10 minutes at a pressure of at least 200 MPa.

3. Pharmaceutical composition according to claim 1, **characterized in that** the tumor cells were treated with hydrostatic pressure for 10 minutes to 2 hours at a pressure of 200-250 MPa.

4. Pharmaceutical composition according to any of claims 1-3, **characterized in that** the apoptotic tumor cells are not necrotic.

5. Pharmaceutical composition according to any of the preceding claims, **characterized in that** the tumor cells were derived from tumor cell lines.

6. Pharmaceutical composition according to any of claims 1-4, **characterized in that** the tumor cells were derived from a tumor isolated from the patient to be treated.

7. Pharmaceutical composition according to any of claims 1-6, **characterized in that** immature dendritic cells were loaded with apoptotic tumor cells which have been treated with high hydrostatic pressure.

8. Pharmaceutical composition according to claim 7, **characterized in that** the immature dendritic cells were obtained by leukapheresis.

9. Pharmaceutical composition according to claim 8, **characterized in that** the immature dendritic cells were obtained by leukapheresis and cultivation in vitro in the presence of cytokines.

10. Method of preparing a pharmaceutical composition according to any of claims 1-9, **characterized in that** immature dendritic cells are loaded with apoptotic tumor cells which have been treated with high hydrostatic pressure and subsequent maturation of the dendritic cells.

11. Method of treatment of a human by cancer vaccination comprising the isolation of immature dendritic cells from the patient and the isolation of tumor cells from the patient whereby said tumor cells are converted to an apoptotic stage by treatment with high hydrostatic pressure, loaded onto the immature dendritic cells and the dendritic cells are matured before the pharmaceutical composition is applied to the patient.
